# EUROPEAN PATENT SPECIFICATION

(11) **EP 4 316 433 B1**
(45) Date of publication and mention of the grant of the patent: **08.04.2026**
(21) Application number: 22796227.1
(22) Date of filing: 29.04.2022
(51) Int. Cl.: A61F 9/007, A61B 17/3205

(54) **PUNCHING-TYPE IRIDOTOMY INSTRUMENT**
IRIDOTOMIE-STANZINSTRUMENT
INSTRUMENT D'IRIDOTOMIE DE TYPE À PERFORATION

(30) Priority: 30.04.2021 KR 20210056618
(43) Date of publication of application: 07.02.2024
(73) Proprietor: Industry-Academic Cooperation Foundation Gyeongsang National University, Jinju-si, Gyeongsangnam-do 52828 (KR); Korea University Research and Business Foundation, Seoul 02841 (KR)
(72) Inventor: KIM, Seong Jae, Jinju-si Gyeongsangnam-do 52728 (KR); EOM, Youngsub, Seoul 05502 (KR)
(74) Representative: Isarpatent
(86) International application number: PCT/KR2022/006217
(87) International publication number: WO 2022/231395

(56) References cited:
- CN-U- 212 996 679
- JP-A- 2007 000 944
- JP-A- H0 647 048
- KR-A- 20100 096 050
- KR-A- 20190 024 157
- KR-A- 20190 024 157
- US-A- 5 683 408
- US-A1- 2020 197 725

## Description

### [Technical Field]

The present invention relates to a punching-type iridotomy instrument, and more particularly, to a punching-type iridotomy instrument which, in iridectomy, increases the size and positioning accuracy iris incision through structural improvement in a perforation method, thereby shortening the surgery time and facilitating the convenience of surgical procedures.

### [Background Art]

Glaucoma refers to an ophthalmic disease caused by abnormalities in the optic nerve, which transmits light entering the eye to the brain, resulting in impaired vision and visual field defects. Examples of risk factors for glaucoma include high intraocular pressure, optic nerve disk hemorrhage, nearsightedness, and thin central corneal thickness.

The aforementioned glaucoma may be broadly classified into open-angle glaucoma and closed-angle glaucoma, depending on the mechanism of impairment of aqueous humor drainage. In the case of closed-angle glaucoma, intraocular pressure increases as the iris around the trabeculae blocks the drainage of aqueous humor through the trabeculae. When acute closed-angle glaucoma is caused by an increase in intraocular pressure, the rapid increase in intraocular pressure causes obvious symptoms such as severe eye pain, headaches, and decreased vision. In addition, if glaucoma is not detected and treated early and properly, permanent vision defects are left behind, and severe glaucoma may even cause blindness.

To treat acute closed-angle glaucoma, iridectomy, which is a surgery that reduces intraocular pressure by creating a small hole in the peripheral iris and draining the aqueous humor, may be used.

Corneal transplantation refers to a surgical procedure that removes the recipient's diseased corneal flap and transplants a clear corneal flap from a donor to treat deterioration in vision caused by a cloudy or edematous cornea. Descemet membrane endothelial keratoplasty (DMEK) or Descemet Stripping automated endothelial keratoplasty (DSAEK) is performed to transplant only the problematic endothelial flap while leaving the normal corneal tissue behind. The iris incision for preventing pupillary blockage may also be performed by the iridectomy.

However, the iridectomy has traditionally been performed by removing a portion of the iris through an incision in the cornea or sclera and cutting out a portion of the iris with surgical scissors. However, when surgical scissors are used, it is difficult to control the exact location and size of the iris incision, and the incision may cause iris hemorrhage. This iris hemorrhage significantly increases the likelihood of failure of glaucoma (closed-angle glaucoma) surgery and corneal transplantation.

As a result, there is an acute need to research and develop an iridotomy instrument that can perform iridectomy while accurately adjusting the size and position of the iris incision, prevent iris hemorrhage by performing optimal iris incision, shorten the surgery time, and facilitate the convenience of the surgical procedure. Surgical punch forceps type instruments for eye surgeries like trabeculectomy are known for example from document US 5683408.

### [Disclosure]

### [Technical Problem]

The present invention has been made in an effort to solve the above-mentioned various problems, and an object of the present invention is to provide a punching-type iridotomy instrument that follows a basic structure and operation mannerism of scissors and accurately sets the size and position of iris incision by improving a structure for forming a hole in a predetermined site of an iris by using a punching method instead of a unique scissor incision method.

Another object of the present invention is to provide a punching-type iridotomy instrument that may more conveniently perform an operation in accordance with a situation by replacing/changing sizes (diameter) and positions of incision (punching) members in consideration of a size and position of iris incision.

### [Technical Solution]

To achieve the above-mentioned objects, the present invention provides a punching-type iridotomy instrument including: a grip manipulation part configured to be manipulated to be opened or closed by a user while being held by the user's hand; an extension shaft extending from the grip manipulation part; and a punching operating unit extending from the extension shaft and configured to perform an operation of forming a hole in a predetermined site of a peripheral portion of an iris in conjunction with an operation of the grip manipulation part performed by the user's manipulation, in which the grip manipulation part includes: a first round shaft extending from a rear side of the extension shaft and having a first finger grip ring formed at an end of the first round shaft and configured to be fitted with the user's thumb; a second round shaft hingedly coupled to a predetermined position on the extension shaft by a pivot pin and having a second finger grip ring formed at an end of the second round shaft and configured to be fitted with the user's ring finger, the second round shaft being configured to implement a rotational operation related to a motion of the ring finger fitted into the second finger grip ring; a spring disposed between the first and second round shafts and configured to exert elasticity for pushing the second round shaft; and a wire configured to connect the second round shaft and the punching operating unit while passing through the extension shaft and operate the punching operating unit in conjunction with a rotational operation of the second round shaft.

In addition, the punching operating unit may include: upper and lower forks extending from a front side of the extension shaft and divided into two pieces to define a spacing interval for accommodating the iris such that the upper and lower forks are respectively positioned outside the iris and inside the iris; a rotary bar hingedly coupled to a tip of the upper fork by a shaft pin and configured to rotate toward the lower fork about the shaft pin by an operation in conjunction with the wire in accordance with a manipulation of the grip manipulation part; and incision members respectively provided at a tip of the lower fork and a tip of the rotary bar and configured to form a hole in the predetermined site of the iris positioned in the spacing interval in conjunction with a rotation of the rotary bar.

Further, the first round shaft may integrally extend from the extension shaft, the first round shaft may have an inclination angle so that the extension shaft is bent downward when the thumb is directed upward in a state in which the thumb and the ring finger are respectively fitted into the first and second finger grip rings, and the inclination angle may be an acute angle larger than 0° and smaller than a right angle based on a length direction of the extension shaft.

In addition, assembling holes may be respectively formed in a tip portion of the lower fork and a tip portion of the rotary bar so that the incision members are coupled to the assembling holes, and the incision member may include: a punch configured to be fitted into the assembling hole, protruding downward from the rotary bar, and configured to form a hole in the predetermined site of the iris in conjunction with the rotation of the rotary bar; and a socket configured to be fitted into the assembling hole of the lower fork to accommodate a distal end of the punch.

Further, a first extension bar may be provided at the tip of the lower fork and configured to increase a length of the lower fork and adjust a position of the socket while being extended from the tip or retracted to the tip, a second extension bar may be provided at the tip of the rotary bar and configured to increase a length of the rotary bar and adjust a position of the punch while being extended from the tip or retracted to the tip, extension/retraction rods may respectively extend from rear ends of the first and second extension bars, and guide holes may be directed inward from the tip of the lower fork and the tip of the rotary bar to allow the extension/retraction rods to be inserted and coupled into the guide holes and to guide extensions and retractions of the extension/retraction rods.

In addition, the punch and the socket may each have a diameter corresponding to a size of iris incision and each be configured to be replaced and used.

Further, a diameter of the punch and a hole of the socket may each have any one of a circular shape, an elliptical shape, and an angular shape.

### [Advantageous Effects]

As can be clearly seen from the above-mentioned description, according to the punching-type iridotomy instrument of the present invention, a hole may be accurately formed at a desired position of the iris based on the user's manipulation of the grip manipulation part and the operation of the punching operating unit in conjunction with the user's manipulation in the state in which the punching operating unit provided in the form of pincers is positioned at a predetermined site of the iris to be incised. This advantage may shorten the surgery time, prevent iris hemorrhage caused by erroneous incision, and prevent complications caused by the iris hemorrhage.

In addition, the incision size of the iris is changed and the incision position of the iris is adjusted by adjusting the length of the lower fork of the punching operating unit and the length of the rotary bar by changing and using the punches and the sockets that constitute the incision member. Therefore, it is possible to obtain the effect of performing the iridectomy customized for each patient and obtaining the best surgical outcome.

Further, through the achievement of the various effects described above, the present invention is a very useful invention that can contribute significantly to the development and revitalization of the medical technology and medical industry related to the treatment of glaucoma (closed-angle glaucoma) or the advancement of iridectomy to prevent pupillary blockage in the process of corneal transplantation (shortening the surgery time, facilitating the convenient surgery, suppressing the occurrence of postoperative complications, etc.).

### [Description of Drawings]

FIGS. 1 and 2 are perspective and front views illustrating a relationship between components of a punching-type iridotomy instrument according to the present invention.
FIG. 3 is a main enlarged cross-sectional view illustrating a relationship between components of a punching operating unit of the punching-type iridotomy instrument according to the present invention.
FIG. 4 is a main enlarged cross-sectional view illustrating a coupling relationship of an incision member of the punching-type iridotomy instrument according to the present invention.
FIGS. 5A, 5B, and 5C are exemplified configuration views illustrating embodiments of the incision member of the punching-type iridotomy instrument according to the present invention.
FIG. 6 is a main enlarged cross-sectional view illustrating another relationship between the components of the punching operating unit of the punching-type iridotomy instrument according to the present invention.
FIGS. 7A to 7F are use state views stepwise illustrating an iris incision process using the punching-type iridotomy instrument according to the present invention.

### [Modes of the Invention]

Hereinafter, exemplary embodiments of the present invention will be described in detail with reference to the accompanying drawings so that those skilled in the art to which the present invention pertains may easily carry out the present invention.

First, in giving reference numerals to constituent elements of the drawings, it should be noted that the same constituent elements will be designated by the same reference numerals, if possible, even though the constituent elements are illustrated in different drawings. In addition, in the description of the present invention, the specific descriptions of publicly known related configurations or functions will be omitted when it is determined that the specific descriptions may obscure the subject matter of the present invention.

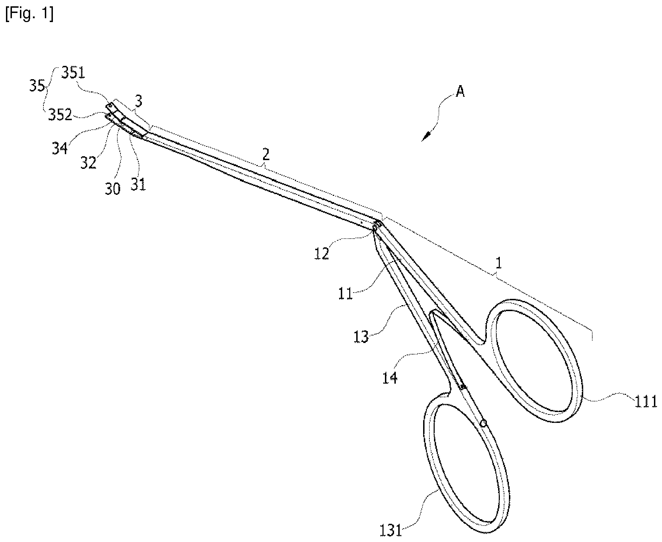

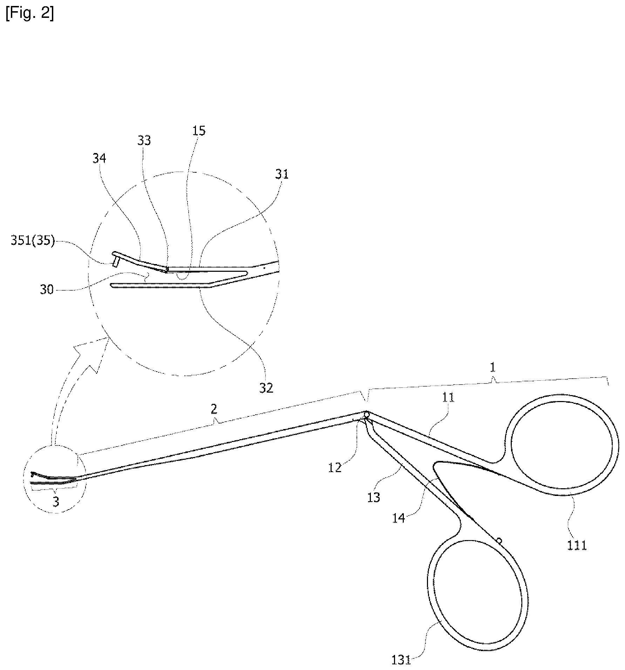

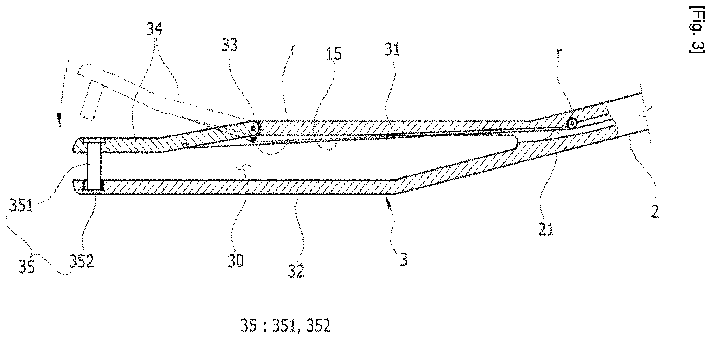

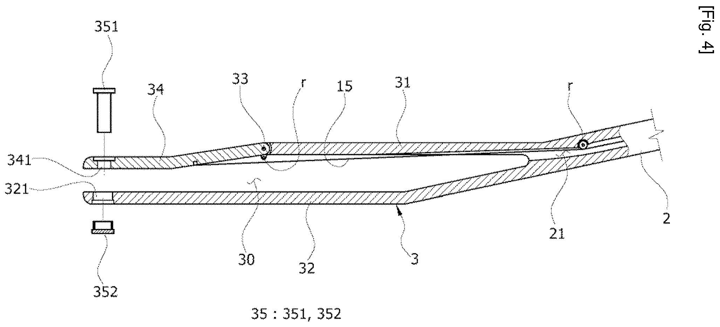

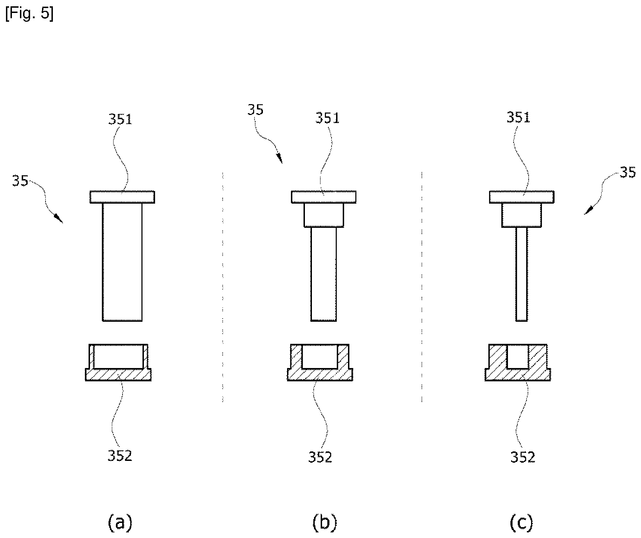

Among the attached drawings, FIGS. 1 and 2 are perspective and front views illustrating a relationship between components of a punching-type iridotomy instrument according to the present invention, FIG. 3 is a main enlarged cross-sectional view illustrating a relationship between components of a punching operating unit of the punching-type iridotomy instrument according to the present invention, FIG. 4 is a main enlarged cross-sectional view illustrating a coupling relationship of an incision member of the punching-type iridotomy instrument according to the present invention, and FIGS. 5A, 5B, and 5C are exemplified configuration views illustrating embodiments of the incision member of the punching-type iridotomy instrument according to the present invention.

As illustrated in FIGS. 1 to 3, a punching-type iridotomy instrument A according to the present invention includes a grip manipulation part 1 configured to be manipulated to be opened or closed by a user while being held by the user's hand, an extension shaft 2 extending from the grip manipulation part 1 by a predetermined length, and a punching operating unit 3 further extending from the extension shaft 2 by a predetermined length and configured to perform an operation of forming a hole in a predetermined site of a peripheral portion of an iris in conjunction with an operation of the grip manipulation part 1 performed by the user's manipulation.

The grip manipulation part 1 is a part manipulated by the user to operate the punching operating unit 3 and control the operation of the punching operating unit 3. The grip manipulation part 1 includes a first round shaft 11 extending rearward from the extension shaft 2 by a predetermined length and having a first finger grip ring 111 formed at an end of the first round shaft 11 and configured to be fitted with the user's thumb, a second round shaft 13 hingedly coupled to a predetermined position on the extension shaft 2 by a pivot pin 12 and having a second finger grip ring 131 formed at an end of the second round shaft 13 and configured to be fitted with the user's ring finger, the second round shaft 13 being configured to implement a rotational operation related to a motion of the ring finger fitted into the second finger grip ring 131, a spring 14 disposed between the first and second round shafts 11 and 13 and configured to exert elasticity for pushing the second round shaft 13, and a wire 15 configured to connect the second round shaft 13 and the punching operating unit 3 while passing through the extension shaft 2 and operate the punching operating unit 3 in conjunction with a rotational operation of the second round shaft 11.

The first round shaft 11 integrally extends from the extension shaft 2. In case that the thumb is directed upward in a state in which the thumb and the ring finger are respectively fitted into the first and second finger grip rings 111 and 131, the extension shaft 2 is bent downward and has an inclination angle θ that provides a grip shape suitable to incise an iris of a patient lying on an operating table. The inclination angle θ may be an acute angle larger than 0° and smaller than a right angle (90°) based on a length direction of the extension shaft 2.

The extension shaft 2 is a part that spaces the punching operating unit 3 from the grip manipulation part 1 so that the user's body part (hand), which holds the grip manipulation part 1, does not come into contact with the patient's surgical site (an eyeball or the like). The extension shaft 2 may be provided in the form of a rod having a predetermined length, and a wire hole 21, through which the wire 15 may pass, may be formed in (formed through) the extension shaft 2 in the length direction.

The punching operating unit 3 is a part that performs an incision operation of forming a small hole in the predetermined site of the peripheral portion of the iris in conjunction with the rotational operation of the second round shaft 13 of the grip manipulation part 1. As illustrated in FIGS. 3 and 4, the punching operating unit 3 includes upper and lower forks 31 and fork 32 extending from a front side of the extension shaft 2 by a predetermined length and divided into two pieces to define a spacing interval 30 for accommodating the iris such that the upper and lower forks 31 and 32 are respectively positioned outside the iris (at the upper side based on the drawing) and inside the iris (at the upper side based on the drawing), a rotary bar 34 hingedly coupled to a tip of the upper fork 31 by a shaft pin 33 and configured to be rotated toward the lower fork 32 about the shaft pin 33 by an operation (pulling operation) in conjunction with the wire 15 in accordance with the manipulation of the grip manipulation part 1, and incision members 35 respectively provided at a tip of the lower fork 32 and a tip of the rotary bar 34 and configured to form a hole in a predetermined site of the iris positioned in the spacing interval 30 in conjunction with the rotation of the rotary bar 34.

Assembling holes 321 and 341 are respectively formed in tip portions of the lower fork 32 and the rotary bar 34 so that the incision members 35 are coupled to the assembling holes 321 and 341.

The incision members 35 include a punch 351 configured to be fitted into the assembling hole 321, protruding downward from the rotary bar 34 by a predetermined length, and configured to form the hole in the predetermined site of the iris in conjunction with the rotation of the rotary bar 34, and a socket 352 configured to be fitted into the assembling hole 341 of the lower fork 32 to accommodate a distal end of the punch 351.

In this case, a diameter of the punch 351 and a hole size of the socket 352, which accommodates the distal end of the punch 351, may vary depending on a size of the hole to be formed in the predetermined site of the iris. As illustrated in FIGS. 5A, 5B, and 5C, the punches 351 having different diameters and the sockets 352 having different holes may be changed and used. Further, although not illustrated in the drawings, the diameter of the punch 351 and the hole of the socket 352 may have various shapes such as a circular, elliptical, quadrangular, hexagonal, or octagonal shape.

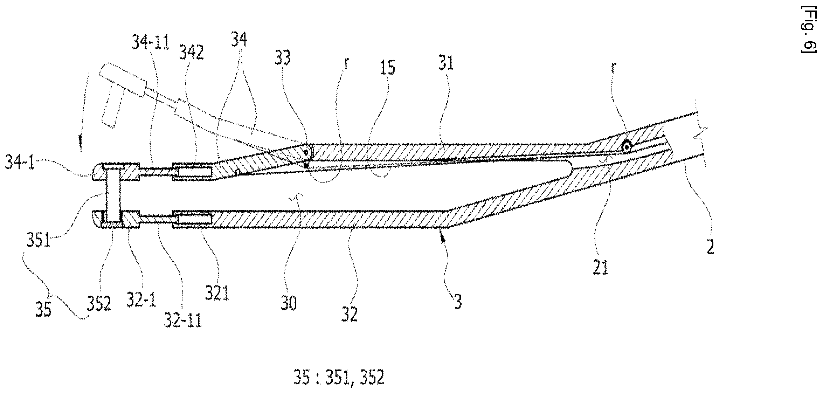

Meanwhile, FIG. 6 is a main enlarged cross-sectional view illustrating another relationship between the components of the punching operating unit of the punching-type iridotomy instrument according to the present invention. The punching operating unit and the rotary bar may vary in configuration in order to adjust a length of the lower fork 32 of the punching operating unit 3 and a length of the rotary bar 34 to adjust the positions of the incision members 35 for each position of an incision site of the iris on which the iridectomy is to be performed.

To this end, as illustrated in FIG. 6, a first extension bar 32-1 is provided at the tip of the lower fork 32 and configured to increase a length of the lower fork 32 and adjust a position of the socket 352 of the punching operating unit 3 while being extended from the tip or retracted to the tip. A second extension bar 34-1 is provided at the tip of the rotary bar 34 and configured to increase a length of the rotary bar 34 and adjust a position of the punch 351 of the punching operating unit 3 while being extended from the tip or retracted to the tip.

In addition, extension/retraction rods 32-11 and 34-11 respectively extend from rear ends of the first and second extension bars 32-1 and 34-1. Guide holes 321 and 341 are directed inward from the tip of the lower fork 32 and the tip of the rotary bar 34 in order to allow the extension/retraction rods 32-11 and 34-11 to be inserted and coupled into the guide holes 321 and 341 and to guide the extensions and retractions of the extension/retraction rods 32-11 and 34-11.

Non-described reference numeral r indicates at least one guide roll having two opposite ends connected to the second round shaft of the grip manipulation part and the rotary bar of the punching operating unit and configured to assist the operation of the wire provided to penetrate the extension shaft in the length direction.

An operation of the punching-type iridotomy instrument A according to the present invention configured as described above will be specifically described below.

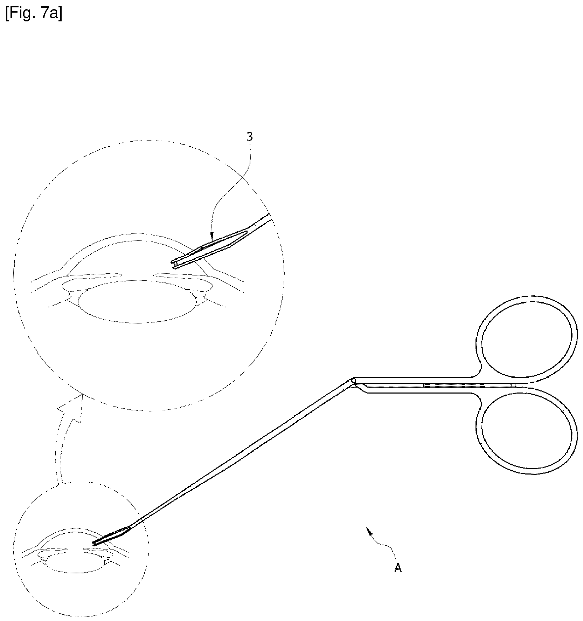

First, FIGS. 7A to 7F are use state views stepwise illustrating an iris incision process using the punching-type iridotomy instrument according to the present invention. As illustrated in the drawings, the punching-type iridotomy instrument A of the present invention may be used for iridectomy performed to prevent pupillary blockage during the cornea transplantation or the treatment of closed-angle glaucoma by reducing intraocular pressure by forming a small hole in a peripheral portion of an iris and draining aqueous humor.

Specifically, as illustrated in FIG. 7A, the punching operating unit 3 is inserted into the cornea (eyeball) through cornea incision in the state in which the rotary bar 34, which is disposed forward of the upper fork 31 of the punching operating unit 3 extending from the front side of the extension shaft 2, is closed as the grip manipulation part 1 of the iridotomy instrument A of the present invention, which is held by the user's hand, is manipulated by the user.

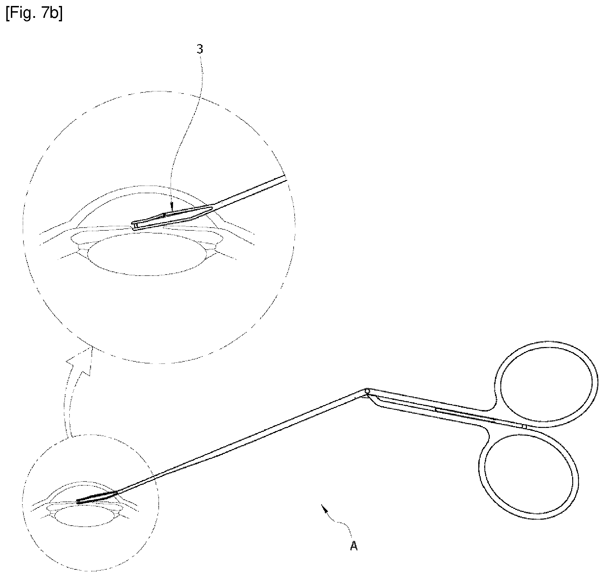

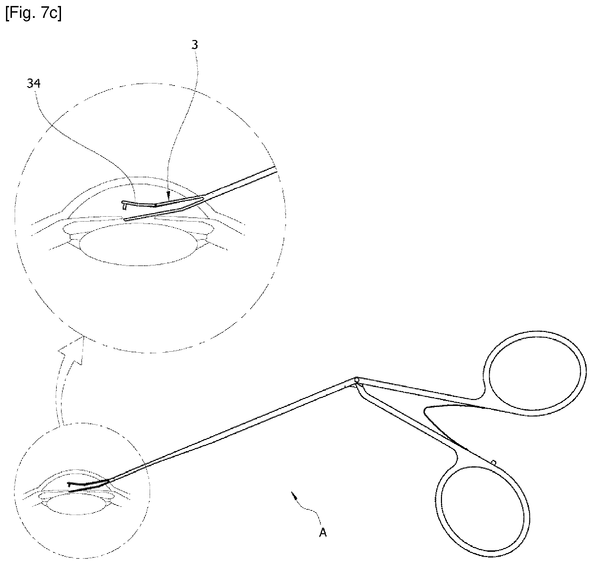

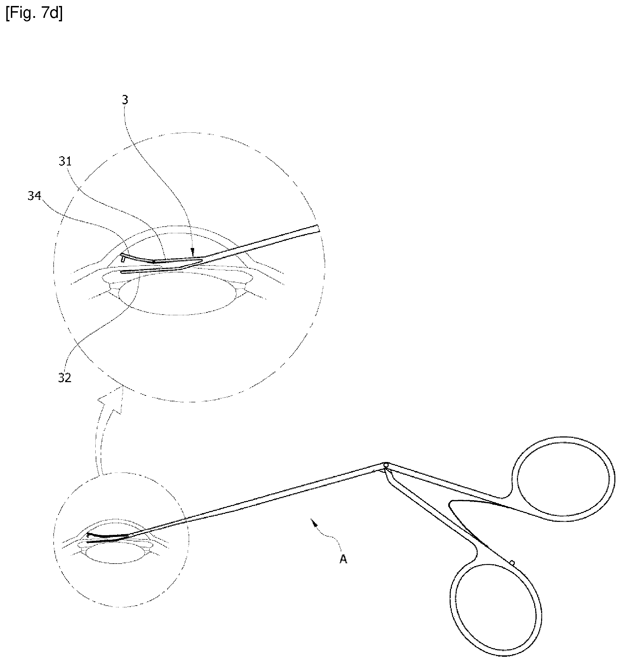

Next, as illustrated in FIG. 7B, the punching operating unit 3 is positioned to an edge of the iris. Thereafter, as illustrated in FIG. 7C, the ring finger fitted into the second finger grip ring 131 of the end of the second round shaft 13 of the grip manipulation part 1 is opened so that the rotary bar 34 is moved away from the lower fork 32 of the punching operating unit 3 by the operation in conjunction with the wire 15 related to the operation of rotating the second round shaft 13. Therefore, in a state in which the iris may enter the spacing interval 30 between the upper fork 31 and the lower fork 32, the punching operating unit 3 enters a predetermined site (position) of the iris to be incised in a state in which the upper fork 31 is positioned outside the iris (at the upper side based on the drawing) and the lower fork 32 is positioned inside the iris (at the lower side based on the drawing), as illustrated in FIG. 7D.

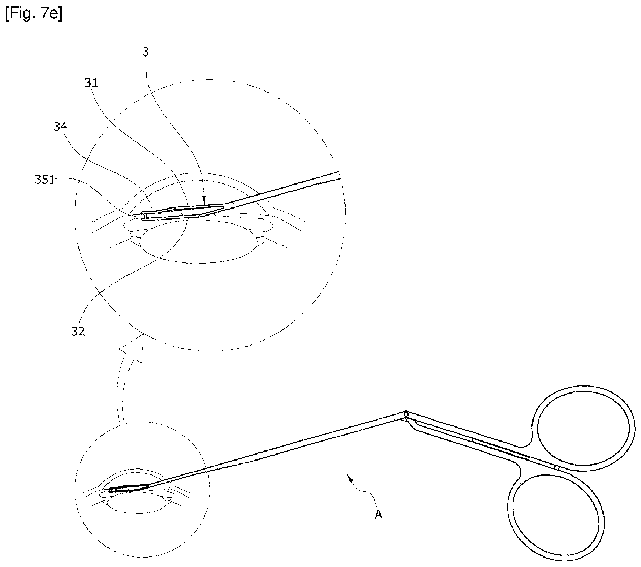

In this state, as illustrated in FIG. 7E, the ring finger is closed, and the rotary bar 34 is rotated toward the lower fork 32 about the shaft pin 33 at the tip of the upper fork 31 by the operation (pulling operation) in conjunction with the wire 15 related to the rotation so that the second round shaft 13 of the second finger grip ring 131, which is fitted with the ring finger, comes into close contact with the first round shaft 11 of the grip manipulation part 1 that is fitted with the thumb. Therefore, a desired site of the peripheral portion of the iris is incised (perforated) by the operation in which the punch 351 of the incision member 35 fitted with the assembling hole 341 of the rotary bar 34 is partially accommodated in the socket 352 of the incision member 35 fitted with the assembling hole 321 of the lower fork 32 while penetrating the iris.

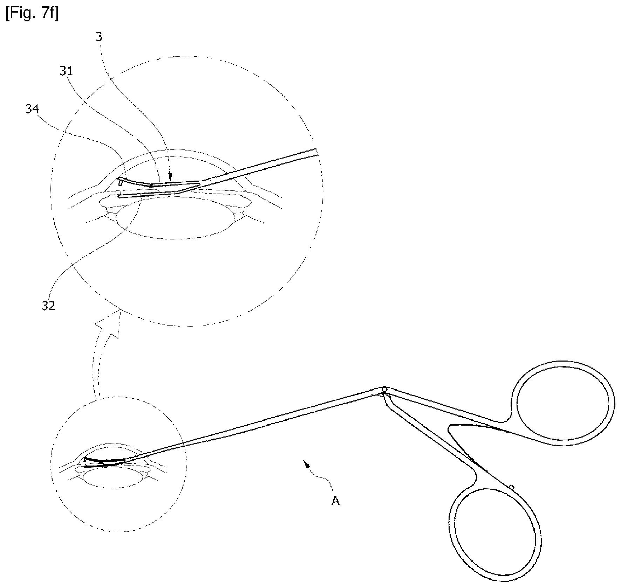

Lastly, after the iris incision is completed as described above, the punching operating unit 3 is moved to a central portion of the cornea in a state in which the ring finger is opened so that the rotary bar 34 is moved away from the lower fork 32, as illustrated in FIG. 7F. Thereafter, the incision of the peripheral portion of the iris is ended by taking out the punching operating unit 3 through the cornea incision in a state in which the ring finger is closed so that the rotary bar 34 is in close contact with the lower fork 32.

As a result, according to the punching-type iridotomy instrument A of the present invention, a hole may be accurately formed at a desired position of the iris based on the user's manipulation of the grip manipulation part 1 and the operation of the punching operating unit 3 in conjunction with the user's manipulation in the state in which the punching operating unit 3 is positioned at a predetermined site of the iris to be incised. This advantage may shorten the surgery time, prevent iris hemorrhage caused by erroneous incision, and prevent complications caused by the iris hemorrhage.

In addition, the incision size of the iris is changed and the incision position of the iris is adjusted by adjusting the length of the lower fork 32 of the punching operating unit 3 and the length of the rotary bar 34 at the tip of the upper fork 31 by changing and using the punches 351 and the sockets 352 that constitute the incision member 35. Therefore, it is possible to obtain the effect of performing the iridectomy customized for each patient and obtaining the best surgical outcome.

## Claims

1. A punching-type iridotomy instrument comprising:
a grip manipulation part (1) configured to be manipulated to be opened or closed by a user while being held by the user's hand;
an extension shaft (2) extending from the grip manipulation part; and
a punching operating unit (3) extending from the extension shaft and configured to perform an operation of forming a hole in a predetermined site of a peripheral portion of an iris in conjunction with an operation of the grip manipulation part performed by the user's manipulation,
wherein the grip manipulation part (1) comprises:
a first round shaft (11) extending from a rear side of the extension shaft (2) and having a first finger grip ring (111) formed at an end of the first round shaft (11) and configured to be fitted with the user's thumb;
a second round shaft (13) hingedly coupled to a predetermined position on the extension shaft (2) by a pivot pin (12) and having a second finger grip ring (131) formed at an end of the second round shaft (13) and configured to be fitted with the user's ring finger, the second round shaft (13) being configured to implement a rotational operation related to a motion of the ring finger fitted into the second finger grip ring (131);
a spring (14) disposed between the first and second round shafts (11,13) and configured to exert elasticity for pushing the second round shaft (13); and
a wire (15) configured to connect the second round shaft (13) and the punching operating unit (3) while passing through the extension shaft (2) and operate the punching operating unit (3) in conjunction with a rotational operation of the second round shaft (13).

2. The punching-type iridotomy instrument of claim 1, wherein the punching operating unit (3) comprises:
upper and lower forks (31,32) extending from a front side of the extension shaft (2) and divided into two pieces to define a spacing interval (30) for accommodating the iris such that the upper and lower forks are respectively positioned outside the iris and inside the iris;
a rotary bar (34) hingedly coupled to a tip of the upper fork (31) by a shaft pin (33) and configured to rotate toward the lower fork (32) about the shaft pin (33) by an operation in conjunction with the wire (15) in accordance with a manipulation of the grip manipulation part (1); and
incision members (35) respectively provided at a tip of the lower fork (32) and a tip of the rotary bar (34) and configured to form a hole in the predetermined site of the iris positioned in the spacing interval in conjunction with a rotation of the rotary bar (34).

3. The punching-type iridotomy instrument of claim 1, wherein the first round shaft integrally extends from the extension shaft,
wherein the first round shaft has an inclination angle so that the extension shaft is bent downward when the thumb is directed upward in a state in which the thumb and the ring finger are respectively fitted into the first and second finger grip rings, and
wherein the inclination angle is an acute angle larger than 0° and smaller than a right angle based on a length direction of the extension shaft.

4. The punching-type iridotomy instrument of claim 2, wherein assembling holes are respectively formed in a tip portion of the lower fork and a tip portion of the rotary bar so that the incision members are coupled to the assembling holes, and
wherein the incision member comprises:
a punch configured to be fitted into the assembling hole, protruding downward from the rotary bar, and configured to form a hole in the predetermined site of the iris in conjunction with the rotation of the rotary bar; and
a socket configured to be fitted into the assembling hole of the lower fork to accommodate a distal end of the punch.

5. The punching-type iridotomy instrument of claim 2, wherein a first extension bar is provided at the tip of the lower fork and configured to increase a length of the lower fork and adjust a position of the socket while being extended from the tip or retracted to the tip,
wherein a second extension bar is provided at the tip of the rotary bar and configured to increase a length of the rotary bar and adjust a position of the punch while being extended from the tip or retracted to the tip,
wherein extension/retraction rods respectively extend from rear ends of the first and second extension bars, and
wherein guide holes are directed inward from the tip of the lower fork and the tip of the rotary bar to allow the extension/retraction rods to be inserted and coupled into the guide holes and to guide extensions and retractions of the extension/retraction rods.

6. The punching-type iridotomy instrument of claim 4, wherein the punch and the socket each have a diameter corresponding to a size of iris incision and are each configured to be replaced and used.

7. The punching-type iridotomy instrument of claim 4, wherein a diameter of the punch and a hole of the socket each have any one of a circular shape, an elliptical shape, and an angular shape.

## Patentansprüche

1. Iridotomie-Instrument vom Stanztyp, umfassend:
einen Griffhandhabungsteil (1), der dazu eingerichtet ist, so gehandhabt zu werden, dass er durch einen Benutzer geöffnet oder geschlossen wird, während er durch die Hand des Benutzers gehalten wird;
einen Verlängerungsschaft (2), der sich von dem Griffhandhabungsteil erstreckt; und
eine Stanzbetätigungseinheit (3), die sich von dem Verlängerungsschaft erstreckt und dazu eingerichtet ist, eine Operation des Bildens eines Lochs an einer zuvor festgelegten Stelle eines peripheren Abschnitts einer Iris in Verbindung mit einer Operation des Griffhandhabungsteils, die durch die Handhabung durch den Benutzer durchgeführt wird, durchzuführen,
wobei der Griffhandhabungsteil (1) umfasst:
einen ersten runden Schaft (11), der sich von einer Rückseite des Verlängerungsschafts (2) erstreckt und einen ersten Fingergriffring (111) aufweist, der an einem Ende des ersten runden Schafts (11) gebildet ist und dazu eingerichtet ist, dass ein Benutzer seinen Daumen hineinschieben kann;
einen zweiten runden Schaft (13), der mittels eines Drehstifts (12) an einer zuvor festgelegten Position an dem Verlängerungsschaft (2) angelenkt ist und einen zweiten Fingergriffring (131) aufweist, der an einem Ende des zweiten runden Schafts (13) gebildet und dazu eingerichtet ist, dass ein Benutzer seinen Ringfinger hineinschieben kann, wobei der zweite runde Schaft (13) dazu eingerichtet ist, eine Drehoperation durchzuführen, die mit einer Bewegung des in den zweiten Fingergriffring (131) eingeschobenen Ringfingers im Zusammenhang steht;
eine Feder (14), die zwischen dem ersten und dem zweiten runden Schaft (11, 13) angeordnet und dazu eingerichtet ist, einem auf den zweiten runden Schaft (13) ausgeübten Druck Elastizität zu verleihen; und
einen Draht (15), der dazu eingerichtet ist, den zweiten runden Schaft (13) und die Stanzbetätigungseinheit (3) zu verbinden, während er durch den Verlängerungsschaft (2) hindurch verläuft, und die Stanzbetätigungseinheit (3) in Verbindung mit einer Drehoperation des zweiten runden Schafts (13) zu betätigen.

2. Iridotomie-Instrument vom Stanztyp nach Anspruch 1, wobei die Stanzbetätigungseinheit (3) umfasst:
eine obere und eine untere Gabel (31, 32), die sich von einer Vorderseite des Verlängerungsschafts (2) erstrecken und in zwei Stücke unterteilt sind, um ein Abstandsintervall (30) zum Aufnehmen der Iris zu definieren, dergestalt, dass die obere und die untere Gabel außerhalb des Iris bzw. innerhalb der Iris positioniert sind;
eine Drehstange (34), die mittels eines Wellenbolzens (33) an einer Spitze der oberen Gabel (31) angelenkt ist und dazu eingerichtet ist, sich durch eine Operation in Verbindung mit dem Draht (15) gemäß einer Handhabung des Griffhandhabungsteils (1) um den Wellenbolzen (33) in Richtung der unteren Gabel (32) zu drehen; und
Inzisionselemente (35), die an einer Spitze der unteren Gabel (32) bzw. einer Spitze der Drehstange (34) bereitgestellt sind und dazu eingerichtet sind, in Verbindung mit einer Drehung der Drehstange (34) ein Loch an der zuvor festgelegten Stelle der Iris, die sich in dem Abstandsintervall befindet, zu bilden.

3. Iridotomie-Instrument vom Stanztyp nach Anspruch 1, wobei sich der erste runde Schaft integral von dem Verlängerungsschaft erstreckt,
wobei der erste runde Schaft einen solchen Neigungswinkel aufweist, dass der Verlängerungsschaft nach unten gebogen ist, wenn der Daumen nach oben gerichtet ist, während der Daumen und der Ringfinger in den ersten bzw. den zweiten Fingergriffring eingeschoben sind, und
wobei der Neigungswinkel ein spitzer Winkel ist, der größer als 0° und kleiner als ein rechter Winkel in Bezug auf eine Längsrichtung des Verlängerungsschafts ist.

4. Iridotomie-Instrument vom Stanztyp nach Anspruch 2, wobei in einem Spitzenabschnitt der unteren Gabel und einem Spitzenabschnitt der Drehstange jeweils Montagelöcher gebildet sind, so dass die Inzisionselemente mit den Montagelöchern gekoppelt sind, und
wobei das Inzisionselement umfasst:
einen Stanzstempel, der dazu eingerichtet ist, in das Montageloch zu passen, von der Drehstange nach unten vorsteht und dazu eingerichtet ist, in Verbindung mit der Drehung der Drehstange ein Loch an der zuvor festgelegten Stelle der Iris zu bilden; und
eine Buchse, die dazu eingerichtet ist, in das Montageloch der unteren Gabel eingepasst zu werden, um ein distales Ende des Stanzstempels aufzunehmen.

5. Iridotomie-Instrument vom Stanztyp nach Anspruch 2, wobei eine erste Verlängerungsstange an der Spitze der unteren Gabel bereitgestellt ist und dazu eingerichtet ist, eine Länge der unteren Gabel zu vergrößern und eine Position der Buchse einzustellen, während sie von der Spitze nach vorn geschoben oder zur Spitze hin zurückgezogen wird,
wobei eine zweite Verlängerungsstange an der Spitze der Drehstange bereitgestellt ist und dazu eingerichtet ist, eine Länge der Drehstange zu vergrößern und eine Position des Stanzstempels einzustellen, während sie von der Spitze nach vorn geschoben oder zur Spitze hin zurückgezogen wird,
wobei sich Ausschub-/Rückziehstangen von hinteren Enden der ersten bzw. der zweiten Verlängerungsstange erstrecken, und
wobei Führungslöcher von der Spitze der unteren Gabel und der Spitze der Drehstange nach innen gerichtet sind, damit die Ausschub-/Rückziehstangen in die Führungslöcher eingeführt und mit diesen gekoppelt werden können und um das Herausschieben und Zurückziehen der Ausschub-/Rückziehstangen zu führen.

6. Iridotomie-Instrument vom Stanztyp nach Anspruch 4, wobei der Stanzstempel und die Buchse jeweils einen Durchmesser haben, der einer Größe der Iris-Inzision entspricht, und jeweils dazu eingerichtet sind, ausgetauscht und verwendet zu werden.

7. Iridotomie-Instrument vom Stanztyp nach Anspruch 4, wobei ein Durchmesser des Stanzstempels und ein Loch der Buchse jeweils eine von einer Kreisform, einer elliptische Form und einer winkeligen Form aufweisen.

## Revendications

1. Instrument d'iridotomie du type à poinçonnage comprenant :
une partie manipulation de poignée (1) conçue pour être manipulée pour être ouverte ou fermée par un utilisateur tout en étant tenue par la main de l'utilisateur ;
une tige d'extension (2) s'étendant à partir de la partie manipulation de poignée ; et
une unité opérationnelle de poinçonnage (3) s'étendant à partir de la tige d'extension et conçue pour effectuer une opération de formation d'un trou dans un site prédéterminé d'une partie périphérique d'un iris conjointement avec une opération de la partie manipulation de poignée effectuée par la manipulation de l'utilisateur,
dans lequel la partie manipulation de poignée (1) comprend :
une première tige ronde (11) s'étendant à partir d'un côté arrière de la tige d'extension (2) et possédant un premier anneau de préhension de doigt (111) formé à une extrémité de la première tige ronde (11) et conçu pour recevoir le pouce de l'utilisateur ;
une seconde tige ronde (13) accouplée de manière articulée à une position prédéterminée sur la tige d'extension (2) par un axe de pivotement (12) et possédant un second anneau de préhension de doigt (131) formé à une extrémité de la seconde tige ronde (13) et conçu pour recevoir l'annulaire de l'utilisateur, la seconde tige ronde (13) étant conçue pour mettre en œuvre une opération de rotation liée à un mouvement de l'annulaire inséré dans le second anneau de préhension de doigt (131) ;
un ressort (14) disposé entre les première et seconde tiges rondes (11, 13) et conçu pour exercer une élasticité pour pousser la seconde tige ronde (13) ; et
un fil (15) conçu pour relier la seconde tige ronde (13) et l'unité opérationnelle de poinçonnage (3) tout en passant à travers la tige d'extension (2) et actionner l'unité opérationnelle de poinçonnage (3) conjointement avec une opération de rotation de la seconde tige ronde (13).

2. Instrument d'iridotomie du type à poinçonnage selon la revendication 1, dans lequel l'unité opérationnelle de poinçonnage (3) comprend :
des fourches supérieure et inférieure (31, 32) s'étendant à partir d'un côté avant de la tige d'extension (2) et divisées en deux parties pour définir un intervalle d'espacement (30) pour recevoir l'iris de telle sorte que les fourches supérieure et inférieure soient respectivement positionnées à l'extérieur de l'iris et à l'intérieur de l'iris ;
une barre rotative (34) accouplée de manière articulée à une pointe de la fourche supérieure (31) par un axe de tige (33) et conçue pour tourner vers la fourche inférieure (32) autour de l'axe de tige (33) par une opération conjointement avec le fil (15) conformément à une manipulation de la partie manipulation de poignée (1) ; et
des éléments d'incision (35) respectivement disposés au niveau d'une pointe de la fourche inférieure (32) et d'une pointe de la barre rotative (34) et conçus pour former un trou dans le site prédéterminé de l'iris positionné dans l'intervalle d'espacement conjointement avec une rotation de la barre rotative (34).

3. Instrument d'iridotomie de type à poinçonnage selon la revendication 1, dans lequel la première tige ronde s'étend d'un seul tenant à partir de la tige d'extension,
dans lequel la première tige ronde présente un angle d'inclinaison de telle sorte que la tige d'extension est pliée vers le bas lorsque le pouce est dirigé vers le haut dans un état dans lequel le pouce et l'annulaire sont respectivement insérés dans les premier et second anneaux de préhension de doigts, et
dans lequel l'angle d'inclinaison est un angle aigu supérieur à 0° et inférieur à un angle droit sur la base d'une direction longitudinale de la tige d'extension.

4. Instrument d'iridotomie de type à poinçonnage selon la revendication 2, dans lequel des trous d'assemblage sont respectivement formés dans une partie de pointe de la fourche inférieure et une partie de pointe de la barre rotative de sorte que les éléments d'incision soient accouplés aux trous d'assemblage, et
dans lequel l'élément d'incision comprend :
un poinçon conçu pour être inséré dans le trou d'assemblage, faisant saillie vers le bas à partir de la barre rotative, et conçu pour former un trou dans le site prédéterminé de l'iris conjointement avec la rotation de la barre rotative ; et
une emboîture conçue pour être insérée dans le trou d'assemblage de la fourche inférieure pour recevoir une extrémité distale du poinçon.

5. Instrument d'iridotomie de type à poinçonnage selon la revendication 2, dans lequel une première barre d'extension est prévue au niveau de la pointe de la fourche inférieure et conçue pour augmenter une longueur de la fourche inférieure et ajuster une position de l'emboîture en étant étendue par rapport à la pointe ou rétractée vers la pointe,
dans lequel une seconde barre d'extension est prévue au niveau de la pointe de la barre rotative et conçue pour augmenter une longueur de la barre rotative et ajuster une position du poinçon en étant étendue par rapport à la pointe ou rétractée vers la pointe,
dans lequel des tiges d'extension/rétraction s'étendent respectivement à partir d'extrémités arrière des première et seconde barres d'extension, et
dans lequel des trous de guidage sont dirigés vers l'intérieur à partir de la pointe de la fourche inférieure et la pointe de la barre rotative pour permettre aux tiges d'extension/rétraction d'être insérées et accouplées dans les trous de guidage et pour guider les extensions et les rétractions des tiges d'extension/rétraction.

6. Instrument d'iridotomie du type à poinçonnage selon la revendication 4, dans lequel le poinçon et l'emboîture ont chacun un diamètre correspondant à une taille d'incision de l'iris et sont chacun conçus pour être remplacés et utilisés.

7. Instrument d'iridotomie du type à poinçonnage selon la revendication 4, dans lequel un diamètre du poinçon et un trou de l'emboîture ont chacun l'une quelconque parmi une forme circulaire, une forme elliptique, ou une forme angulaire.
